# EUROPEAN PATENT SPECIFICATION

(11) **EP 2 335 917 B1**
(45) Date of publication and mention of the grant of the patent: **30.04.2014**
(21) Application number: 09816213.4
(22) Date of filing: 25.09.2009
(51) Int. Cl.: A61L 27/00, A61L 27/04, B05D 1/18, B05D 1/28, B82Y 30/00, B82Y 40/00, C09D 5/44, C08F 292/00, C25D 11/16, C25D 11/26, A61L 27/34

(54) **POLYMER BRUSH COMPOSITE AND METHOD FOR PRODUCING SAME**
POLYMERBÜRSTENVERBUNDSTOFF UND VERFAHREN ZU SEINER HERSTELLUNG
COMPOSITE DE BROSSE POLYMÈRE ET PROCÉDÉ DE PRODUCTION ASSOCIÉ

(30) Priority: 26.09.2008 JP 2008247361
(43) Date of publication of application: 22.06.2011
(73) Proprietor: National University Corporation Tokyo Medical and Dental University, Bunkyo-ku Tokyo 113-8510 (JP)
(72) Inventor: HANAWA, Takao, Tokyo 113-8510 (JP); TSUTSUMI, Yusuke, Tokyo 113-8510 (JP); NAM, Kwangwoo, Tokyo 113-8510 (JP); KISHIDA, Akio, Tokyo 113-8510 (JP); KOBAYASHI, Hisatoshi, Tsukuba-shi Ibaraki 305-0047 (JP); YOSHIKAWA, Chiaki, Tsukuba-shi Ibaraki 305-0047 (JP)
(74) Representative: von Kreisler Selting Werner
(86) International application number: PCT/JP2009/066680
(87) International publication number: WO 2010/035799

(56) References cited:
- WO-A1-94/11118
- WO-A2-02/056021
- WO-A2-2004/114371
- WO-A2-2006/132694
- JP-A- 8 244 155
- JP-A- 2003 168 682
- JP-A- 2005 319 679
- JP-A- 2006 255 811
- JP-A- 2008 177 283
- JP-T- 2007 527 605

## Description

### TECHNICAL FIELD

The present invention relates to a polymer brush composite and a method for producing the same.

### BACKGROUND ART

A variety of composite materials that include an organic polymer compound and an inorganic material such as a metal have been proposed. For example, a composite material has been disclosed that includes a substrate such as a metal and a polymer compound having one end immobilized on the surface of the substrate (for example, see Japanese Patent Application Laid-Open (JP-A) No. 2006-177914). Further, a composite material has been disclosed that includes a substrate such as a metal and a biocompatible material layer, which are adhered to each other via a binder layer (for example, see JP-A No. 2007-260247).

WO 2004/114371 discloses a compound use to form a self-assembled monolayer, a layer structure and a semiconductor component having a layer structure and a method for producing a layer structure.

WO 02/056021 discloses polymer brushes for immobilizing molecules to a surface or substrate having improved stability.

WO 2006/132694 discloses a process for the modification of a substrate so as to form an ultra hydrophobic surface on the substrate.

WO 94/11118 discloses a method for modifying a surface to develop a microscopically smooth, biocompatible surface.

### DISCLOSURE OF THE INVENTION

### TECHNICAL PROBLEM

However, in the composite material described in JP-A No. 2006-177914 or JP-A No. 2007-260247, since the organic polymer compound is immobilized on the inorganic material at only one end of the organic polymer compound, the binding between the organic polymer compound and the inorganic inorganic material is insufficient. In addition, it has not been possible to form a composite material surface at which the organic polymer compound is uniformly present in the inorganic material in such a manner that the organic polymer compound has an orientation.

It is an object of the present invention to provide a composite material that exhibits favorable binding between an organic polymer compound and an inorganic material and has a surface at which all or a part of the organic polymer compound is uniformly present in the inorganic material in such a manner that the organic polymer compound has an orientation, and a method for producing the composite material.

### SOLUTION TO PROBLEM

A first aspect of the present invention is a polymer brush composite including a substrate, a solid layer disposed on the substrate and containing at least one of a metal or a metal oxide, and a plurality of polymer chains penetrating the solid layer in such a manner that one end of each of the polymer chains is disposed on an upper side of the substrate and the other end of each of the polymer chains is exposed at a surface of the solid layer on an opposite side of the solid layer to the substrate.

The polymer chains preferably have a ratio (Mw/Mn) of weight average molecular weight (Mw) to number average molecular weight (Mn) of 1.0 or more and 1.5 or less.

A second aspect of the present invention is a method for producing the polymer brush composite of the invention, the method including a polymer brush forming step of forming a plurality of polymer chains on a substrate, one end of each of the polymer chains being disposed on the substrate, and a solid layer forming step of forming a solid layer containing at least one of a metal or a metal oxide in a region in which the polymer chains are not immobilized on the surface of the substrate that has the polymer chains formed thereon, by applying a voltage to the substrate in a medium, wherein in the solid layer forming step, a metal oxide layer is formed by anodic oxidation.

The polymer brush forming step preferably includes a step of forming a plurality of polymerization initiation points on the substrate and a step of polymerizing a polymerizable monomer using the plurality of polymerization initiation points as starting points.

### ADVANTAGEOUS EFFECT OF INVENTION

According to the present invention, it is possible to provide a composite material that exhibits favorable binding between an organic polymer compound and an inorganic material and that has a surface at which all or a part of the organic polymer compound is uniformly present in the inorganic material in such a manner that the organic polymer compound has an orientation, and a method for producing the composite material.

### BRIEF DESCRIPTION OF DRAWINGS

Fig. 1 shows schematic views showing one example of a method for forming polymer chains on a substrate according to the Example of the present invention.
Fig. 2 shows schematic cross-sectional views illustrating one example of the structure of a polymer brush and a polymer brush composite according to the Example of the present invention.
Fig. 3 shows charts indicating the results of XPS according to the Example of the present invention, in which the upper sections show the results before anodic oxidation treatment and the lower sections show the results after anodic oxidation treatment.
Fig. 4 shows charts indicating the results of AES according to the embodiment of the present invention, in which the left chart indicates the results before anodic oxidation treatment and the right chart indicates the results after anodic oxidation treatment.
Fig. 5 shows fluorescent microscopic photographs showing the evaluation results of cell adhesion according to the Example of the present invention.

### BEST MODE FOR CARRYING OUT THE INVENTION

A polymer brush composite according to the present invention includes a substrate, a solid layer disposed on the substrate and containing at least one of a metal or a metal oxide, and a plurality of polymer chains penetrating the solid layer in a state in which the polymer chains are extended in such a manner that one end of each of the polymer chains is disposed on the upper side of the substrate and the other end of each of the polymer chains is exposed at the surface of the solid layer at the opposite side of the solid layer to the substrate.

In such a configuration, one end of each of the polymer chains is buried in the solid layer, thereby enabling the formation of a composite material that exhibits favorable binding between the organic polymer chains and the inorganic material (the solid layer) and has a surface at which the organic polymer compound is uniformly present in the inorganic material in such a manner that the organic polymer compound has an orientation.

The substrate is not specifically limited as long as the solid layer containing at least one of a metal or a metal oxide can be disposed and one end of the polymer chain can be disposed. Preferably, the substrate has electric conductivity. Preferably, at least the surface of the substrate on which the polymer chains are disposed is smooth. Examples of the substrate may include a metal, a metal oxide, an alloy, and a semiconductor.

Examples of the metal may include gold, silver, copper, platinum, titanium, aluminum, nickel, and zirconium. Examples of the metal oxide may include titanium oxide, zirconium oxide, chromium oxide, and aluminum oxide. Examples of the alloy may include titanium alloy, cobalt-chromium alloy, stainless steel, and gold alloy. Examples of the semiconductor include silicon.

The substrate used in the present invention can be appropriately selected according to the purpose of the polymer brush composite. For example, from the viewpoint of biocompatibility, the substrate is preferably selected from titanium, zirconium, cobalt-chromium alloy, stainless steel, and gold alloy, and more preferably selected from titanium, zirconium, and gold.

The solid layer contains at least one of a metal or a metal oxide. Examples of the metal and the metal oxide for the solid layer may include metals such as titanium and zirconium, and oxides thereof.

In the present invention, the metal and the metal oxide for the solid layer can be appropriately selected according to the purpose of the polymer brush composite. From the viewpoint of formationability of the solid layer, a metal that is the same as at least one of metals contained in the substrate or an oxide thereof is preferable.

The polymer forming the polymer chains is not specifically limited as long as one end of the polymer chain can be disposed on the substrate. The polymer may be an addition polymerization polymer or a condensation polymerization polymer. Specific examples of the polymer may include vinyl polymers, epoxy polymers, polyester polymers, polyurethane polymers, polyamide polymers, polycarbonate polymers, polyurea polymers, polyether polymers, and polyimide polymers. The polymer is preferably a vinyl polymer, a polyester polymer, a polyamide polymer, or a polyether polymer.

In the present invention, the polymer chains can be appropriately selected according to the purpose of the polymer brush composite.

The polymer forming the polymer chains in the present invention can be synthesized using commonly used monomer(s). Examples of the monomers used to form an addition polymerization polymer may include acrylate esters, acryl amide, methacrylate esters, methacryl amide, vinyl esters, vinyl ether, and olefins. Polyurethane polymers can be obtained by polymerization of a diol compound and a diisocyanate compound. Polyester polymers can be obtained either by condensation polymerization of a diol compound and a dicarboxylic acid compound or by ring-opening polymerization of a lactone. Polyamide polymers can be obtained by condensation polymerization of a diamine compound and a dicarboxylic acid compound, by condensation polymerization of an aminocarboxylic acid compound, or by ring-opening polymerization of a lactam.

Polymer synthesis conditions in the present invention can be appropriately selected according to the monomer to be used.

The polymer forming the polymer chains in the present invention is preferably a vinyl polymer from the viewpoint of general versatility.

Examples of the method for synthesizing a vinyl polymer include living radical polymerization, living anionic polymerization, and living cationic polymerization. In the present invention, living radical polymerization is preferable from the viewpoint of industrial application and facilitation.

The weight average molecular weight of the polymer chains in the present invention is not specifically limited. For example, the weight average molecular weight thereof may be in a range of from 400 to 10,000,000, and preferably in a range of 10,000 to 200,000.

The polymer chains in the present invention have a ratio (Mw/Mn) of weight average molecular weight (Mw) to number average molecular weight (Mn) of preferably from 1.0 to 1.5, and more preferably from 1.0 to 1.3. When the ratio Mw/Mn is within the above ranges, the length difference between the individual polymer chains is smaller, so that the surface of the polymer brush composite is in such a state that the polymer compound and the inorganic material more uniformly coexist on the surface thereof.

The polymer chains penetrate the solid layer. Since the plurality of polymer chains penetrating the solid layer, the end of each polymer chain that is not the end disposed on the upper side of the substrate is exposed at the surface of the solid layer (the surface of the solid layer on an opposite side of the solid layer to the substrate), whereby the inorganic material and the organic polymer compound uniformly coexist on the surface of the solid layer containing the inorganic material.

There is no specific limitation to the manner in which the polymer chains penetrate the solid layer. From the viewpoint of the uniform coexistence of the inorganic material and the organic polymer compound, preferably, the polymer chains penetrate the solid layer in a state in which the polymer chains are extended. The state in which the polymer chains are extended defined herein means that the state in which the polymer chains are not in an aggregation state, and the definition thereof includes not only a state in which the polymer chains are completely extended, but also a state in which the polymer chains are extended with thermodynamic fluctuations.

Furthermore, the state in which the polymer chains penetrate the solid layer in such a manner that one end of each of the polymer chains is exposed at the surface of the solid layer means the state in which the one end of a polymer chains is buried in the solid layer and at least a part of the other end thereof is protruded from the surface of the solid layer at the opposite side of the solid layer to the substrate.

In the present invention, in the polymer brush forming step of forming the polymer chains on the substrate, one end of the each polymer chain is immobilized on the substrate. Examples of the manner of the immobilization may include immobilization by covalent bonding, immobilization by ionic bonding, immobilization by hydrophobic interaction, and immobilization by electrodeposition. Preferred is covalent bonding from the viewpoint of adhesion of the polymer compound.

The immobilizing density of the polymer chains on the substrate is preferably no less than a immobilizing density at which another polymer chain exists in the radius of inertia of one polymer chain and a mutually repellent force begins to act between the polymer chains, and no more than a immobilizing density at which the polymer chains are closely packed in a state in which the polymer chains are completely extended.

Specifically, the immobilizing density thereof is preferably from 0.001 to 1.0 chain/nm², and more preferably from 0.05 to 0.8 chains/nm². When the polymer chains are immobilized on the substrate at such a high density as in the above ranges, the interaction between the polymer chains allows the polymer chains to be easily form a state in which the polymer chains are extended, thereby making more uniform the coexisting state of the inorganic material and the organic polymer compound on the surface of the solid layer.

The polymer brush composite according to the present invention has a conventionally unknown, novel structure, and the organic polymer compound is uniformly present on the surface of the inorganic material (the solid layer) in such a manner that the organic polymer compound has an orientation. Thereby, the polymer brush composite of the present invention can exhibit new properties that cannot be achieved by the inorganic material alone or the organic polymer compound alone, and possesses properties similar to those of a semiconductor.

The polymer brush composite of the present invention can be applied to a variety of purposes by appropriately combining a metal or a metal oxide forming the solid layer with a polymer forming the polymer chains. For example, the polymer brush composite can be applied to high-speed electron exchange between a polymer functional group and a metal, as well as to the development of high-sensitivity sensors.

Furthermore, when protein adsorption inhibitory effects and non-cell-adherent properties of the polymer brush composite surface are considered and a metal material widely used in vivo is combined, the polymer brush composite of the present invention can be particularly preferably used as a biocompatible material.

Hereinafter, a detailed explanation will be given of a method for producing a polymer brush composite according to the present invention.

The polymer brush composite producing method according to the present invention includes a polymer brush forming step of forming a plurality of polymer chains on a substrate, one end of each of the polymer chains being disposed on the substrate, and a solid layer forming step of forming a solid layer containing at least one of a metal or a metal oxide on the surface of the substrate that has the polymer chains formed thereon, by applying a voltage to the substrate in a medium, wherein tin the solid layer forming step, a metal oxide layer is formed by anodic oxidation.

With the structure thus described, the polymer brush composite can be efficiently produced.

The substrate and the polymer chains described above have the same definitions as the substrate and the polymer chains of the polymer brush composite previously described respectively, and preferable embodiments thereof are the same as those of the substrate and the polymer chains of the polymer brush composite previously described respectively.

In the present invention, there is no specific limitation to the method for disposing one end of each polymer chain on the substrate. Examples thereof include a method in which a plurality of functional groups that can be bonded to an organic compound are disposed on a substrate surface and the functional groups are bonded to polymer molecules, and a method in which a plurality of polymerization initiation points are formed on a substrate, and a polymer is obtained by polymerization starting from the polymerization initiation points.

The method for disposing, on the substrate surface, the functional groups that can bond to an organic compound or the polymerization initiation points, may be, for example, a method in which the substrate surface is treated with a compound (a coupling agent) having a functional group capable of reacting with and binding to an organic material and a functional group capable of reacting with and binding to an inorganic material in its molecule.

The coupling agent to be used is not specifically limited as long as it is the compound that has, in its molecule, a functional group capable of reacting with and binding to an organic material and a functional group capable of reacting with and biding to an inorganic material. Examples of the functional group capable of reacting with and binding to an inorganic material may include a trialkoxysilyl group, a trihalogenosilyl group, an azido group, and a pyridinium group. Examples of the functional group capable of reacting with and binding to an organic material may include a haloalkyl group, an amino group, a hydroxy group, a mercapto group, a carboxyl group, a thiol group, and a specific reaction including avidin-biotin reaction. The coupling agent may have a coupling group that connects the functional group capable of reacting with and binding to an inorganic material to the functional group capable of reacting with and binding to an organic material.

In the present invention, the coupling agent is preferably a silane coupling agent from the viewpoint of reactivity with the substrate.

Specific examples of the silane coupling agent include 6-(2-bromo-2-isobutyryloxy) hexyl triethoxy silane (BHTE), 6-(2-bromo-2-isobutyryloxy) hexyl diethoxy silane (BHDE), and 6-(2-bromo-2-isobutyryloxy) hexyl monoethoxy silane (BHME).

In the present invention, the polymer brush forming step preferably includes a step of forming a plurality of polymerization initiation points on the substrate and a step of polymerizing a polymerizable monomer using the plurality of polymerization initiation points as starting points. In this way, the polymer brush can be formed more efficiently.

Herein, as the step of forming the polymerization initiation points on the substrate, the method in which the substrate surface is treated with the above-mentioned coupling agent can be suitably applied.

The step of polymerizing the polymerizable monomer using the polymerization initiation points as starting points can be carried out under conditions that are appropriately selected according to the kind of the polymerization initiation points and the polymerizable monomer to be used.

In the present invention, at the polymer brush forming step, one end of each polymer chain is immobilized on the substrate. The immobilizing density of the polymer chains on the substrate is preferably from 0.001 to 1.0 chains/nm², and more preferably from 0.05 to 0.8 chains/nm². When the immobilizing density of the polymer chains is in the above ranges, the interaction between adjacent polymer chains allows the polymer chains to easily form a state in which the polymer chains are extended, so that the polymer brush composite can be produced more efficiently.

The immobilizing density of the polymer chains on the substrate can be controlled, for example, by appropriately selecting conditions for the surface treatment of the substrate using the coupling agent.

In the solid layer forming step in the present invention, the solid layer containing at least one of a metal or a metal oxide is formed on the surface of the substrate that has the polymer chains formed thereon (particularly, in a region in which the polymer chains are not immobilized), by applying a voltage to the substrate in a medium. In this way, the solid layer can be formed in the state in which the polymer chains having one end disposed on the substrate penetrate the solid layer.

The medium is preferably an aqueous medium containing water. The aqueous medium is not specifically limited as long as it is a water-containing medium. The aqueous medium can be formed by further including an organic solvent, an inorganic salt, and the like, according to needs.

In the present invention, by applying a voltage to the substrate, the solid layer containing at least one of a metal or a metal oxide is formed on the surface of the substrate that has the polymer chains formed thereon.

The voltage to be applied and the method for applying the voltage can be appropriately selected according to the metal and the metal oxide forming the solid layer. For example, a voltage of from 1 to 20 V can be applied in such a manner that the voltage is maintained to be constant.

In the present invention, when forming the solid layer containing a metal oxide on the substrate, a metal oxide derived from a metal forming the substrate is disposed on the substrate by anodic oxidation using the substrate as an anode. In this case, the metal oxide is mainly disposed on the region in which the polymer chains are not immobilized, whereby the solid layer is formed in such a manner that the polymer chains penetrate the metal oxide layer.

In the present invention, when forming the solid layer containing a metal on the substrate, a metal derived from metal ions present in the aqueous medium can be disposed on the substrate by adding metal ions in the aqueous medium and using the substrate as a cathode. In this case, the metal is mainly disposed on the region in which the polymer chains are not immobilized, whereby the solid layer is formed in such the manner that the polymer chains penetrate the metal layer.

In the present invention, the solid layer forming step is preferably performed in such a manner that the non-immobilized end of each polymer chain immobilized on the substrate in the polymer brush forming step is exposed at the surface of the solid layer on an opposite side of the solid layer to the substrate. This can provide the state in which the inorganic material and the organic polymer compound uniformly coexist on the solid layer surface.

In the solid layer forming step, the voltage to be applied to the substrate and the voltage applying method according to the polymer chains immobilized on the substrate are appropriately selected, thereby providing the state in which the non-immobilized end of each polymer chain is exposed at the surface of the solid layer on an opposite side of the solid layer to the substrate.

### EXAMPLES

Hereinafter, the present invention is described in further detail below by reference to Examples. It should be however noted that the present invention is not limited to those Examples. In addition, "%" is based on mass unless otherwise specifically stated.

### EXAMPLE 1

In a THF solution containing 1% of aqueous ammonia and 1% of 6-(2-bromo-2-isobutyryloxy) hexyl triethoxy silane (BHE), a metal titanium plate as a substrate was soaked at room temperature for 12 hours. Thereafter, the substrate was washed with THF and methanol, and the substrate that had been subjected to surface treatment with BHE was obtained.

A methanol solution containing 25 mM of Cu(I)Br and 63 mM of bipyridine was stirred for 30 minutes to completely dissolve the Cu(I)Br. In this methanol solution, 4.5 M of 2-hydroxyethyl methacrylate (HEMA) and 22.5 mM of ethyl 2-bromoisobutyrate (EBIB) were added and mixed. The obtained solution was placed in a glass tube containing the titanium plate surface-treated with BHE and the grass tube was sealed. The sealed glass tube was shaked for 90 minutes in a water bath at 40°C to carry out polymerization reaction, thereby obtaining a substrate having polymer chains (PolyHEMA) derived from HEMA formed on the surface thereof (a polymer brush).

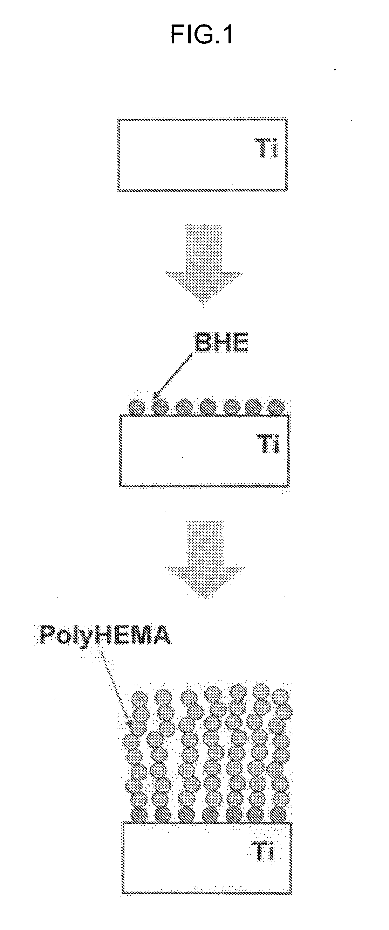

Fig. 1 schematically shows that the HEMA-derived polymer chains (PolyHEMA), each having one end immobilized on the titanium plate, is formed on the titanium plate.

The polymer solution obtained by the above polymerization reaction was diluted with DMF and the weight average molecular weight was measured using GPC. The result is that Mw was 20,300. In addition, polydispersity index (PDI: Mw/Mn) was calculated and the value of Mw/Mn obtained was 1.28.

The GPC was performed using the CCP & 8020 series high-speed liquid chromatography (manufactured by Toso Co., Ltd.) and as a standard sample, polyethylene glycol.

With respect to the above-obtained substrate having the polymer chains formed thereon, the surface state was observed using an automatic ellipsometer (DVA-36L3 manufactured by Mizojiri Optical Co. Ltd.), and the results are shown in Table 1. Table 1 indicates that the polymer chains are uniformly formed on the titanium surface.

**Table 1**

| Layer Thickness of Polymer Chains (nm) | Density (chain/nm²) | Average Distance between Polymer Chains (nm) |
|---|---|---|
| 16.4 | 0.56 | 1.4 |

Next, the above-obtained substrate having the polymer chains formed thereon was subjected to anodic oxidation using a potentiostat (HA-501 G manufactured by Hokuto Denko Corp.) to electrodeposit titanium oxide on the substrate. In this way, a polymer brush composite was obtained.

Using a saturated calomel electrode (SCE) as a reference electrode and a platinum electrode with platinum-black as a counter electrode, the anodic oxidation treatment was carried out for 30 seconds in a 0.1 M sodium sulfate solution by applying voltages of 1 V_{SCE}, 5 V_{SCE}, and 10 V_{SCE}, respectively.

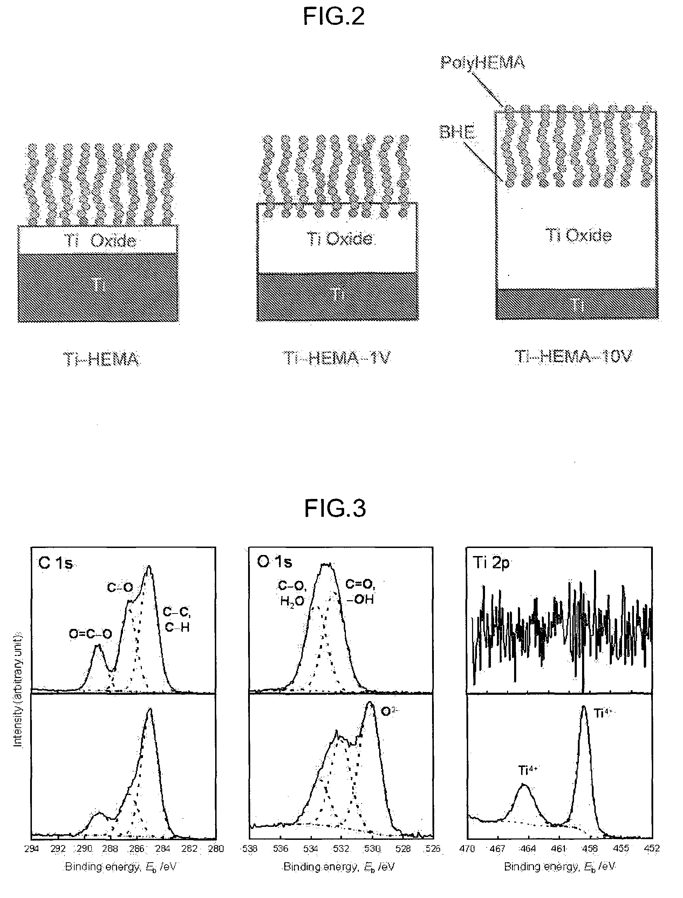

In Fig. 2, one example of an estimated structure of the substrate having the polymer chains formed thereon (Ti-HEMA) before the anodic oxidation treatment is shown at the left side.

Further, in Fig. 2, a schematic cross-sectional view illustrating one example of an estimated structure of a polymer brush composite (Ti-HEMA-1V) to which the voltage of 1 V_{SCE} has been applied is shown at the center, and a schematic cross-sectional view illustrating one example of an estimated structure of a polymer brush composite (Ti-HEMA-10V) to which the voltage of 10 V_{SCE} has been applied is shown at the right side.

With respect to each of the polymer brush composites (Ti-HEMA-1V, Ti-HEMA-5V, and Ti-HEMA-10V) to which the voltages of 1, 5, and 10 V_{SCE} were applied, a contact angle with water was measured using a contact angle meter. Additionally, with respect to comparative samples (Ti-1V, Ti-5V, and Ti-10V) obtained by subjecting a metal titanium plates having no polymer chains formed thereon to anodic oxidation in the same manner as in the above-described process, and the substrate (Ti-HEMA) having the polymer chains formed thereon but not having been subjected to the anodic oxidation, were each subjected to the measurement of a contact angle with water. Table 2 shows the results.

**Table 2**

| Sample | Contact Angle (°C) |
|---|---|
| Ti-1V | 83 |
| Ti-5V | 86 |
| Ti-10V | 87 |
| Ti-HEMA | 47 |
| Ti-HEMA-1V | 67 |
| Ti-HEMA-5V | 62 |
| Ti-HEMA-10V | 63 |

Table 2 indicates that the polymer brush composite of the present invention exhibited properties intermediate between the organic compound and the inorganic material. It is also found that even with application of any voltage of 1, 5, and 10 V, the surface states were similar.

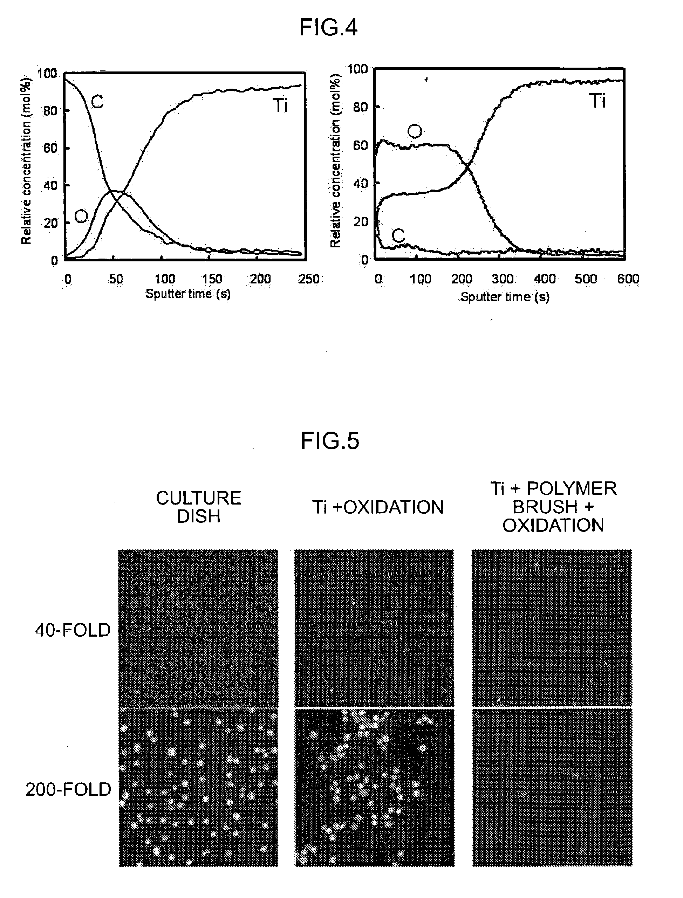

Fig. 3 shows the results of X-ray photoelectron spectroscopy (XPS) of the polymer brush composite and Fig. 4 shows the results of Auger electron spectroscopy (AES) of the polymer brush composite. The XPS was carried out using the SSX 100 (manufactured by SSI technologies, Inc.) by a usual method and the AES was carried out using the JAMP-7100 (manufactured by JEOL Ltd.) by a usual method.

The spectra in the upper sections of Fig. 3 indicate that, before the anodic oxidation treatment, the substrate surface had no titanium thereon and was covered with organic polymer chains. The spectra in the lower sections of Fig. 3 indicate that, as a result of the anodic oxidation treatment, the organic polymer compound and titanium coexisted on the surface of the polymer brush composite.

The left-side chart of Fig. 4 indicates that, before the anodic oxidation treatment, the substrate surface was covered with the organic polymer chains. The right-sided chart of Fig. 4 indicates that, as a result of the anodic oxidation treatment, the organic polymer compound and titanium oxide coexisted on the surface of the polymer brush composite. Furthermore, as the sputtering proceeded, the amount of the organic polymer compound decreased, whereby it is found that the polymer chains were buried in the titanium oxide layer.

In other words, it is found that, in the polymer brush composite of the present invention, the polymer chains are in the state in which they penetrate the titanium oxide layer (the solid layer) and one end of each polymer chain is exposed at the surface of the solid layer.

Next, cell adhesion of the polymer brush composite of the present invention was evaluated. On the surface of the above-obtained polymer brush composite that has the polymer chains exposed thereon, HeLa cells were cultured for 6 hours under normal culture conditions (culture medium: 10% FCS-added DMEM, 37°C, and 5% CO₂). In observation using a fluorescent microscope, most of the HeLa cells did not adhere to the surface of the polymer brush composite, as shown on the right side of Fig. 5. The upper sections of Fig. 5 show observation results in a 40-fold magnification, and the lower sections thereof show observation results in a 200-fold magnification.

Meanwhile, using a common culture dish and a comparative sample of an anodically oxidized titanium substrate, HeLa cells were cultured for 6 hours under the same conditions and then observed respectively by the fluorescent microscope. The left-sided column of Fig. 5 shows the result of the culture dish, and the center column of Fig. 5 shows the result of the comparative sample, respectively. As seen in the photographs on the left-sided column and the center column of Fig. 5, adhesion of the HeLa cells were found on both of the culture dish and the comparative sample.

As described hereinabove, it is found that the polymer brush composite according to the present invention is a composite material that exhibits good adhesion between an organic polymer compound and an inorganic material and has the surface where the organic polymer compound and the inorganic material uniformly coexist. It is also found that the surface of the polymer brush composite of the present invention is non-cell-adherent.

## Claims

1. A polymer brush composite, comprising: a substrate, a solid layer disposed on the substrate and containing at least one of a metal or a metal oxide, and a plurality of polymer chains penetrating the solid layer in such a manner that one end of each of the polymer chains is disposed on an upper side of the substrate and the other end of each of the polymer chains is exposed at a surface of the solid layer on an opposite side of the solid layer to the substrate.

2. The polymer brush composite according to claim 1, wherein the polymer chains have a ratio (Mw/Mn) of weight average molecular weight (Mw) to number average molecular weight (Mn) of from 1.0 to 1.5.

3. A method for producing a polymer brush composite according to claims 1 or 2, the method comprising:
a polymer brush forming step of forming a plurality of polymer chains on a substrate, one end of each of the polymer chains being disposed on the substrate; and
a solid layer forming step of forming a solid layer containing at least one of a metal or a metal oxide on a surface of the substrate that has the polymer chains formed thereon, by applying a voltage to the substrate in a medium, wherein, in the solid layer forming step, a metal oxide layer is formed by anodic oxidation.

4. The method for producing a polymer brush composite according to claim 3, wherein the polymer brush forming step includes a step of forming a plurality of polymerization initiation points on the substrate and a step of polymerizing a polymerizable monomer using the plurality of polymerization initiation points as starting points.

## Patentansprüche

1. Polymerbürstenverbundstoff, umfassend: ein Substrat, eine feste Schicht, die sich auf dem Substrat befindet und ein Metall und/oder ein Metalloxid enthält, und eine Vielzahl von Polymerketten, die in einer solchen Weise in die feste Schicht eindringen, dass sich jeweils ein Ende der Polymerketten auf einer oberen Seite des Substrats befindet und das jeweils andere Ende der Polymerketten auf einer Oberfläche der festen Schicht auf einer dem Substrat entgegengesetzten Seite der festen Schicht exponiert ist.

2. Polymerbürstenverbundstoff gemäß Anspruch 1, wobei die Polymerketten ein Verhältnis (Mw/Mn) von Gewichtsmittel des Molekulargewichts (Mw) zu Zahlenmittel des Molekulargewichts (Mn) von 1,0 bis,1,5 aufweisen.

3. Verfahren zur Herstellung eines Polymerbürstenverbundstoffs gemäß Anspruch 1 oder 2, wobei das Verfahren Folgendes umfasst:
einen Schritt des Bildens von Polymerbürsten, bei dem eine Vielzahl von Polymerketten auf einem Substrat gebildet werden, wobei sich jeweils ein Ende der Polymerketten auf dem Substrat befindet; und
einen Schritt des Bildens einer festen Schicht, bei dem eine feste Schicht, die ein Metall und/oder ein Metalloxid enthält, auf einer Oberfläche des Substrats, die die darauf gebildeten Polymerketten aufweist, durch Anlegen einer Spannung an das Substrat in einem Medium gebildet wird, wobei in dem Schritt des Bildens einer festen Schicht eine Metalloxidschicht durch anodische Oxidation gebildet wird.

4. Verfahren zur Herstellung eines Polymerbürstenverbundstoffs gemäß Anspruch 3, wobei der Schritt des Bildens von Polymerbürsten einen Schritt des Bildens einer Vielzahl von Polymerisationsstartpunkten auf dem Substrat und einen Schritt des Polymerisierens eines polymerisierbaren Monomers unter Verwendung der Vielzahl von Polymerisationsstartpunkten als Startpunkte umfasst.

## Revendications

1. Composite de brosse polymère comportant : un substrat, une couche solide disposée sur le substrat et contenant au moins l'un d'un métal ou d'un oxyde métallique, et une pluralité de chaînes polymères pénétrant dans la couche solide de telle manière qu'une extrémité de chacune des chaînes polymères est disposée sur un côté supérieur du substrat et que l'autre extrémité de chacune des chaînes polymères est exposée au niveau d'une surface de la couche solide sur un côté de la couche solide opposé au substrat.

2. Composite de brosse polymère selon la revendication 1, dans lequel les chaînes polymères présentent un rapport (Mw/Mn) de la masse moléculaire moyenne en poids (Mw) à la masse poids moléculaire (Mn) moyenne en nombre allant de 1,0 à 1,5.

3. Procédé de production d'un composite de brosse polymère selon les revendications 1 ou 2, le procédé comportant :
une étape de formation de brosse polymère consistant à former une pluralité de chaînes polymères sur un substrat, une extrémité de chacune des chaînes polymères étant disposée sur le substrat, et
une étape de formation d'une couche solide consistant à former une couche solide contenant au moins l'un d'un métal ou d'un oxyde métallique sur une surface du substrat qui présente les chaînes polymères formées sur elle, en appliquant une tension au substrat dans un milieu, dans lequel, dans l'étape de formation de la couche solide, une couche d'oxyde métallique est formée par oxydation anodique.

4. Procédé de production d'un composite de brosse polymère selon la revendication 3, dans lequel l'étape de formation de la brosse polymère comprend une étape consistant à former une pluralité de points d'initialisation de la polymérisation sur le substrat et une étape de polymérisation d'un monomère polymérisable en utilisant la pluralité des points d'initialisation de la polymérisation comme points de départ.
